# EUROPEAN PATENT SPECIFICATION

(11) **EP 2 471 778 B1**
(45) Date of publication and mention of the grant of the patent: **08.05.2019**
(21) Application number: 10826849.1
(22) Date of filing: 29.10.2010
(51) Int. Cl.: C09B 5/62, H01L 51/00, C07D 221/18

(54) **PERYLENE TETRACARBOXYDIIMIDE DERIVATIVE**
PERYLEN-TETRACARBOXYDIIMID-DERIVAT
DÉRIVÉ DE PÉRYLÈNE TÉTRACARBOXYDIIMIDE

(30) Priority: 29.10.2009 JP 2009249127
(43) Date of publication of application: 04.07.2012
(73) Proprietor: Dainichiseika Color & Chemicals Mfg. Co., Ltd., Chuo-ku Tokyo 103-8383 (JP)
(72) Inventor: HIRATA, Naoki, Tokyo 103-8383 (JP); KONO, Hisao, Tokyo 103-8383 (JP); SAIKATSU, Hiroaki, Tokyo 103-8383 (JP); OGUMA, Naomi, Tokyo 103-8383 (JP)
(74) Representative: Hartz, Nikolai
(86) International application number: PCT/JP2010/069277
(87) International publication number: WO 2011/052719

(56) References cited:
- EP-A1- 0 061 088
- WO-A1-2009/118742
- CN-A- 101 353 349
- DE-A1- 3 235 526
- DE-A1- 3 413 418
- DE-A1- 3 434 059
- JP-A- 51 067 328
- JP-A- 57 074 361
- JP-A- 57 176 046
- JP-A- 60 089 485
- US-A- 4 156 757
- US-A- 4 156 757
- BRIAN A GREGG AND RUSSELL A CORMIER: "Doping Molecular Semiconductors: n-Type Doping of a Liquid Crystal Perylene Diimide", JOURNAL OF THE AMERICAN CHEMICAL SOCIETY, ACS PUBLICATIONS, US, vol. 123, no. 32, 1 January 2001 (2001-01-01), pages 7959-7960, XP008154037, ISSN: 0002-7863, DOI: 10.1021/JA016410K [retrieved on 2001-07-21]
- GREGG, B.A. ET AL.: 'Doping molecular emiconductors. n-Type doping of a liquid crystalperylene diimide' JOURNAL OF THE AMERICAN CHEMICAL SOCIETY vol. 123, no. 32, 2001, pages 7959 - 7960, XP008154037
- CHE, Y. ET AL.: 'Enhancing One-Dimensional Charge Transport through Intermolecular n-Electron Delocalization: Conductivity Improvement for Organic Nanobelts' JOURNAL OF THE AMERICAN CHEMICAL SOCIETY vol. 129, no. 20, 2007, pages 6354 - 6355, XP008154038
- SAMUDRALA, R. ET AL.: 'Synthesis of a non- cationic, water-soluble perylenetetracarboxylic diimide and its interactions with G-quadruplex- forming DNA' BIOORGANIC & MEDICINAL CHEMISTRY vol. 15, no. 1, 2007, pages 186 - 193, XP008154043
- SHIRMAN, E. ET AL.: 'Stable Aromatic Dianion in Water' JOURNAL OF PHYSICAL CHEMISTRY B vol. 112, no. 30, 2008, pages 8855 - 8858, XP008154048
- HERRIKHUYZEN, V.J. ET AL.: 'Synthesis of n-Type Perylene Bisimide Derivatives and Their Orthogonal Self-Assembly with p-Type Oligo (p-phenylene vinylene)s' JOURNAL OF THE AMERICAN CHEMICAL SOCIETY vol. 126, no. 32, 2004, pages 10021 - 10027, XP008154034
- FISCHER, C. M. ET AL.: 'Microstructured gold/ Langmuir-Blodgett film/gold tunneling junctions' APPLIED PHYSICS LETTERS vol. 66, no. 24, 1995, pages 3331 - 3, XP008154040

## Description

### Technical Field

This invention relates to a perylene tetracarboxylic diimide derivative having specific substituent groups.

### Background Art

As organic semiconductor materials for use in the formation of organic thin-film solar cells, organic transistors, organic photoconductors or the like, a variety of pigment derivatives such as phthalocyanine pigments, perylene pigments and azo pigments have been employed conventionally. Among these, perylene pigment derivatives have excellent semiconductor characteristics, and perylene derivatives with parts of the perylene skeletons thereof being cyanated to have improved semiconductor characteristics (Patent Document 1), perylene derivatives with durability improved by the introduction of fluorinated alkyl groups (Patent Document 2) and like have been reported.

### Prior Art Documents

### Patent Documents

Patent Document 1: JP-A-2007-527114
Patent Document 2: JP-A-2008-524846

US 4156757 A, EP 0061088 A1, DE 3434059 A1, and Gregg et al, Journal of the American Chemical Society, 2001, 123(32), 7959-7960 disclose perylene tetracarboxylic diimide derivatives.

### Disclosure of the Invention

### Problem to Be Solved by the Invention

Although the conventionally-known various perylene pigment derivatives are excellent in properties such as light resistance and heat resistance compared with dyes or the like having absorption in the visible light range, no perylene pigment derivatives have heretofore been obtained with characteristics sufficient for their use as organic semiconductor materials.

### Means for Solving the Problem

With a view to making improvements in organic semiconductor materials for use in organic thin-film solar cells, organic transistors, organic photoconductors or the like, the present inventors have conducted research and have found that the perylene tetracarboxylic diimide derivative represented by the below-described formula (3), has excellent characteristics, leading to the present invention.

Described specifically, the present invention provides the perylene tetracarboxylic diimide derivative represented by the below-described formula (3) :

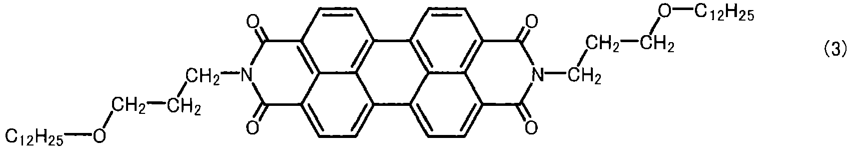

### Advantageous Effects of the Invention

According to the present invention, there is provided a perylene tetracarboxylic diimide derivative which exhibits superb semiconductor characteristics, is excellent in durability when formed into thin films, has high solubility to organic solvents, and can be applied as useful organic semiconductor material.

### Brief Description of the Drawings

FIG. 1 is an infrared absorption spectrum of an exemplary compound 1.
FIG. 2 is an infrared absorption spectrum of an exemplary compound 2.

### Modes for Carrying out the Invention

The present invention will next be described in further detail based on best modes for carrying it out. The perylene tetracarboxylic diimide derivative according to the present invention can be synthesized by known processes. For example, perylene tetracarboxylic acid anhydride may be reacted with a corresponding amine in a high-boiling organic solvent such that its specific substituent groups, which characterize the present invention, are introduced. As an alternative, the perylene tetracarboxylic diimide derivative according to the present invention can also be obtained by once converting perylene tetracarboxylic diimide into its potassium salt and then reacting the potassium salt with the corresponding alkyl halide to introduce its specific substituent groups.

As amine component usable upon synthesis of the perylene tetracarboxylic diimide derivative according to the present invention, the below-described amine component can be mentioned. A usable example includes 3-(n-dodecyloxy)-n-propylamine.

As perylene tetracarboxylic acid anhydrides upon synthesis of the perylene tetracarboxylic diimide derivative according to the present invention, the below-described perylene tetracarboxylic acid anhydride can be mentioned. A usable example includes unsubstituted 3,4:9,10-perylene tetracarboxylic acid anhydride.

When using a perylene tetracarboxylic diimide derivative in an organic solar cell, organic photoconductor, organic transistor or the like, it has generally been an often common practice to deposit it on a substrate under a high vacuum. As one of major reasons for this conventional practice, most of the conventionally-known perylene tetracarboxylic diimide derivatives are insoluble in organic solvents.

In contrast, the perylene tetracarboxylic diimide derivative according to the present invention is relatively soluble in organic solvents unlike the conventional derivatives. Therefore, the derivative according to the present invention can use a purification method such as column chromatography upon its production and, when applied as organic semiconductor materials, can be dissolved in an organic solvent and coated by a method such as spin coating to form coating films (thin films). In Table 2 to be described subsequently herein, there are shown the results when attempts were made to form thin films by using exemplary compounds (Exemplary Componds 2 to 17 are reference compounds) of formula (1) : 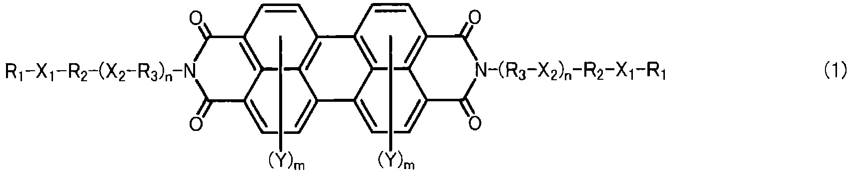 and the compound of the present invention (Exemplary Compound 1) in various organic solvents. Their dissolution characteristics in organic solvents indicate that they can spectacularly improve the application of perylene tetracarboxylic diimide derivatives as organic semiconductor materials and he like.

Their semiconductor characteristics and their solubility to the organic solvents will hereinafter be described. Specific examples of the perylene tetracarboxylic diimide derivatives each of which has specific substituent groups, will be described. It is to be noted that these specific examples will be shown in Table 1 in which their groups and atoms corresponding to the individual signs in the above-described formula (1) are specified, respectively. Production processes of these compounds are similar to Example 1 to be described subsequently herein.

**Table 1 Examples of the Perylene Tetracarboxylic Diimide Derivative Represented by the Formula (1)**

| Exemplary compound | R₁ | X₁ | R₂ | R₃ | X₂ | n | Y | m |
|---|---|---|---|---|---|---|---|---|
| 1 | n-C₁₂H₂₅ | O | -C₃H₆- | - | - | 0 | - | 0 |
| 2 | n-C₄H₉ | O | -C₃H₆- | - | - | 0 | - | 0 |
| 3 | n-C₆H₁₃ | O | -C₃H₆- | - | - | 0 | - | 0 |
| 4 | n-C₈H₁₇ | O | -C₃H₆- | - | - | 0 | - | 0 |
| 5 | sec-C₈H₁₇ | O | -C₃H₆- | - | - | 0 | - | 0 |
| 6 | iso-C₆H₁₃ | O | -C₃H₆- | - | - | 0 | - | 0 |
| 7 | tert-C₄H₉ | O | -C₃H₆- | - | - | 0 | - | 0 |
| 8 | n-C₄H₉ | O | -C₂H₄- | -C₂H₄- | O | 1 | - | 0 |
| 9 | n-C₁₂H₂₅ | O | -C₂H₄- | -C₂H₄- | O | 1 | - | 0 |
| 10 | n-C₁₂H₂₅ | O | -CH(CH₃)CH₂- | -CH(CH₃)CH₂- | O | 1 | - | 0 |
| 11 | n-C₆H₁₃ | O | -CH(CH₃)CH₂- | -CH(CH₃)CH₂- | O | 1 | - | 0 |
| 12 | n-C₄H₉ | S | -C₃H₆- | - | - | 0 | - | 0 |
| 13 | n-C₁₂H₂₅ | S | -C₃H₆- | - | - | 0 | - | 0 |
| 14 | n-C₁₂H₂₅ | Se | -C₃H₆- | - | - | 0 | - | 0 |
| 15 | n-C₁₂H₂₅ | O | -C₃H₆- | - | - | 0 | CN | 2 |
| 16 | n-C₁₂H₂₅ | O | -C₃H₆- | - | - | 0 | F | 4 |
| 17 | n-C₁₂H₂₅ | O | -C₂H₄- | -C₂H₄- | O | 1 | Cl | 2 |

The perylene tetracarboxylic diimide derivative of the present invention as exemplified above is relatively soluble, for example, in organic solvents such as those to be described below. Illustrative are halogenated hydrocarbon solvents such as chloroform, dichloroethane, chlorobenzene, dichlorobenzene, trichlorobenzene and chloronaphthalene, ketone solvents such as acetone, methyl ethyl ketone and methyl isobutyl ketone, ester solvents such as ethyl acetate and butyl acetate, ether solvents such as diethyl ether, dioxane and tetrahydrofuran, aromatic hydrocarbon solvents such as toluene, xylene and ethylbenzene, aprotic polar solvents such as sulfolane, N,N-dimethylformamide, N-methyl-2-pyrrolidone and dimethyl sulfoxide, and the like. The perylene tetracarboxylic diimide derivative according to the present invention can be formulated into solutions of suitable concentrations by using such organic solvents as described above either singly or in combination. With the perylene tetracarboxylic diimide derivative according to the present invention, it is, therefore, possible to apply a solution coating process, such as spin coating, which has not heretofore been applicable to the conventional derivatives, and to readily form uniform thin films by the process. Moreover, the perylene tetracarboxylic diimide derivative according to the present invention has sufficient semiconductor characteristics so that the thin films so formed function as good organic semiconductor thin films, for example, in organic thin-film solar cells, organic transistors, organic photoconductors or the like.

To confirm the solubility of the perylene tetracarboxylic diimide derivative of the present invention to organic solvents , a determination was made, as to the above-exemplified compounds 1 to 17, based on the results of the formation of thin films on silicon substrates by spin coating while using the respective organic solvents. The results are shown in Table 2. As a result, it has been confirmed that the perylene tetracarboxylic diimide derivative of the present invention exhibits good solubility to organic solvents and a solution coating process such as spin coating can be applied.

**Table 2 Solubility of Exemplary Compounds 1 to 17 to Organic Solvents**

| Exemplary compound | Chloroform | Toluene | Tetrahydrofuran | N-methylpyrrolidone |
|---|---|---|---|---|
| 1 | A | D | D | D |
| 2 | A | B | D | C |
| 3 | A | C | D | D |
| 4 | A | C | D | D |
| 5 | A | B | C | B |
| 6 | A | B | C | B |
| 7 | A | B | D | B |
| 8 | A | C | D | C |
| 9 | B | C | D | C |
| 10 | B | D | D | C |
| 11 | A | C | D | C |
| 12 | A | C | C | B |
| 13 | B | D | D | C |
| 14 | B | D | D | D |
| 15 | A | B | C | B |
| 16 | A | B | C | B |
| 17 | A | B | C | C |

| | | | | |
|---|---|---|---|---|
| <Evaluation standards> Determined based on the results of the formation of a thin film on a silicon substrate by spin coating. A: A uniform thin film can be formed. B: Formation of a uniform thin film is feasible by heating the solution. C: Formation of a thin film is feasible only on a part of a substrate by heating the solution. D: Difficult to form a thin film. | | | | |

### Examples

The present invention will hereinafter be described more specifically based on examples. In the individual examples, the exemplary compounds 1 to 17 described above in Table 1 were synthesized, respectively.

### Example 1

### (Synthesis of the exemplary compound 1)

Perylene tetracarboxylic acid anhydride (3.92 g) and 3-(n-dodecyloxy)-n-propylamine (9.72 g) were dispersed in imidazole (40 g), followed by stirring at 160°C for 4 hours under a nitrogen gas stream. The reaction mixture was allowed to cool, and was then filtered. The resulting filter cake was washed with a dilute solution of hydrochloric acid in methanol and with water in this order. Subsequently, the filter cake was dried to afford, as the exemplary compound 1, N,N'-bis(3-(n-dodecyloxy)-n-propyl-3,4:9,10-perylene tetracarboxylic diimide represented by the below-described formula (3) (yield: 70%).

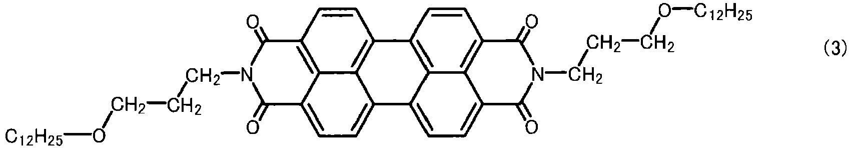

The results of respective analyses of the exemplary compound 1 obtained as described above are as will be described below. Based on these analysis results, the exemplary compound 1 has been identified to be one having the above-described structure.
Melting point: around 330°C (decomposed) Elemental analysis data (calculated values in parentheses): C; 76.88%(76.92%), H; 8.30%(8.37%), N; 3.25%(3.32%)

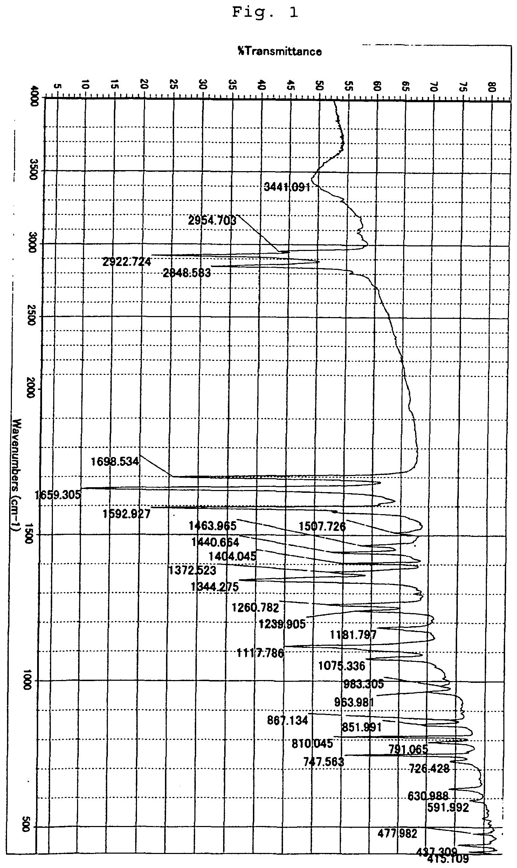

An infrared absorption spectrum is shown in FIG. 1.

### Example 2 (Reference)

### (Synthesis of the exemplary compound 2)

In a similar manner as in Example 1 except that 3-(n-dodecyloxy)-n-propylamine (9.72 g) employed in Example 1 was changed to 3-(n-butyloxy)-n-propylamine (5.24 g), N,N'-bis(3-(n-butyloxy)-n-propyl)-3,4:9,10-perylene tetracarboxylic diimide was afforded as the exemplary compound 2 (yield: 80%). The results of respective analyses of the synthesized exemplary compound 2 are as will be described below. Based on these analysis results, the exemplary compound 2 has been identified to be the above-described compound.
Melting point: around 340°C (decomposed) Elemental analysis data (calculated values in parentheses): C; 73.81%(73.77%), H; 6.15%(6.19%), N; 4.49%(4.53%)

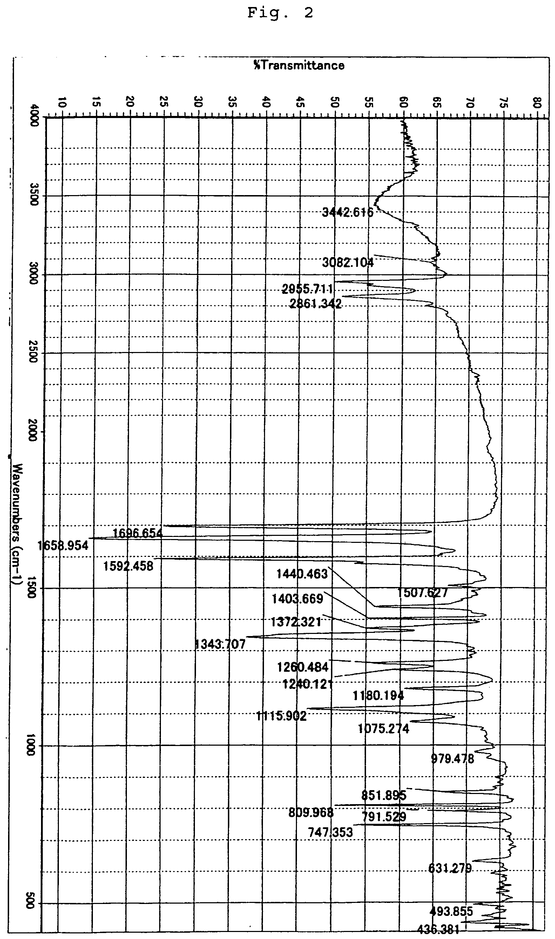

An infrared absorption spectrum is shown in FIG. 2.

### Examples 3 to 17 (Reference)

In a similar manner as in Examples 1 and 2, the exemplary compounds 3 to 17 the structures of which are shown above in Table 1 were synthesized, respectively. The yields, melting points, and the maximum absorption wavelengths in ultraviolet/visible absorption spectra of the afforded respective compounds are shown in Table 3. With respect to the exemplary compounds 1 and 2 afforded in Examples 1 and 2, their maximum absorption wavelengths were also measured, and the results are shown along with their yields and melting points. The ultraviolet/visible absorption spectra of the exemplary compounds 3 to 17 were substantially the same as those of the exemplary compounds 1 and 2.

The yields, maximum absorption wavelengths and melting points of the exemplary compounds 1 to 17 synthesized in the respective examples will be described below in Table 3.

**Table 3 Yields and Characteristics of Respective Exemplary Compounds**

| Example | Exemplary compound | Yield (%) | Maximum absorption wavelength (nm) | Melting point (decomposition point)(°C) |
|---|---|---|---|---|
| 1 | 1 | 80 | 488,525 | Around 340 |
| 2 | 2 | 70 | 488,525 | Around 330 |
| 3 | 3 | 72 | 489,525 | Around 325 |
| 4 | 4 | 83 | 488,525 | Around 320 |
| 5 | 5 | 70 | 488,525 | Around 320 |
| 6 | 6 | 75 | 487,525 | Around 310 |
| 7 | 7 | 90 | 488,525 | Around 300 |
| 8 | 8 | 66 | 488,526 | Around 280 |
| 9 | 9 | 78 | 488,524 | Around 300 |
| 10 | 10 | 55 | 488,525 | Around 265 |
| 11 | 11 | 60 | 489,526 | Around 285 |
| 12 | 12 | 38 | 488,525 | Around 330 |
| 13 | 13 | 45 | 488,525 | Around 290 |
| 14 | 14 | 53 | 488,525 | Around 280 |
| 15 | 15 | 60 | 490,528 | Around 300 |
| 16 | 16 | 55 | 490,531 | Around 330 |
| 17 | 17 | 40 | 492,533 | Around 320 |

Fabrication examples of organic semiconductors using the perylene tetracarboxylic diimide derivative of the present invention will hereinafter be described.

### Referential Example 1

### Fabrication of an organic thin-film transistor with the exemplary compound 1 [N,N'-bis(3-(n-dodecyloxy)-n-propyl)-3,4:9,10-perylene tetracarboxylic diimide]

Provided first was a silicon substrate having on a surface thereof a silicon oxide film (thickness: 200 nm) to be used as a gate insulator layer. An organic semiconductor thin film formed of the exemplary compound 1 afforded in Example 1 was formed to a thickness of 30 nm on the silicon oxide film by vacuum evaporation (deposition rate: 2 nm/sec). Through a shadow mask, a pattern of gold electrodes (30 nm) was formed as source/drain electrodes, whereby top-contact, organic thin-film transistors were fabricated. The channel length and channel width at that time were 100 µm and 2,000 µm, respectively.

Each transistor obtained as described above was measured for drain voltage and drain current at different gate voltages. As a result, a pronounced saturation region was observed on a drain current-drain voltage curve, so that the transistor was indicated to operate as a field effect transistor having typical n-type characteristics. The electron mobility calculated from the curve was 3.3 × 10⁻² cm²/Vs, and the threshold voltage value was 15 V.

### Referential Example 2

The exemplary compound 1 employed in Referential Example 1 was dissolved at a concentration of 0.25% in chloroform. Using the resultant solution, an organic semiconductor thin film was formed on a silicon substrate, which was provided with an oxide film and had ITO electrodes, by a spin coater (1,500 rpm, 40 seconds), followed by reduced-pressure drying at 140°C for 1 hour in a vacuum.

Each transistor obtained as described above was measured for transistor characteristics as in Referential Example 1. As a result, the transistor was indicated to operate as a field effect transistor having typical n-type characteristics. Further, the electron mobility and threshold voltage value were 0.40 cm²/Vs and 16 V. It has, therefore, been confirmed that a transistor is obtained with better semiconductor characteristics when a thin film is formed by spin coating than when it is formed by vacuum evaporation as in Referential Example 1.

### Referential Example 3

In this referential example, the exemplary compound 2 [N,N'-bis(3-(n-butyloxy)-n-propyl)-3,4:9,10-perylen e tetracarboxylic diimide] afforded in Example 2 was used in place of the exemplary compound 1 employed in Referential Example 1.

A top-contact, organic thin-film transistor having an organic semiconductor layer formed of the exemplary compound 2 was fabricated as in Referential Example 1. The transistor was indicated to operate as a field effect transistor having typical n-type characteristics. Further, the electron mobility and threshold voltage value were 3.8 × 10⁻³ cm²/Vs and 10 V.

### Industrial Applicability

According to the present invention, there is provided a perylene tetracarboxylic diimide derivative which exhibits superb semiconductor characteristics, is excellent in durability when formed into thin films, and can be applied as useful organic semiconductor material. As the perylene tetracarboxylic diimide derivative provided by the present invention exhibits solubility to organic solvents, it can be formed into thin films or the like by a solution coating process and is expected to find extremely high practical utility.

## Claims

1. A perylene tetracarboxylic diimide derivative represented by the following formula (3):

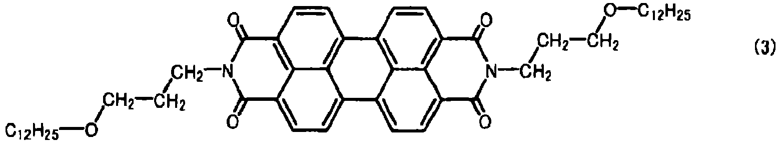

## Patentansprüche

1. Perylentetracarbonsäurediimidderivat der folgenden Formel (3):

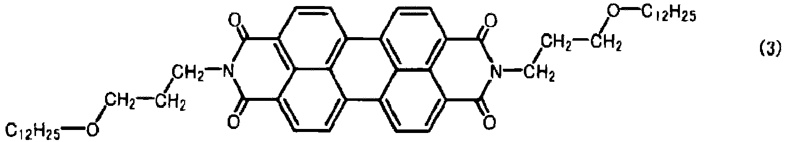

## Revendications

1. Dérivé de pérylène-diimide tétracarboxylique représenté par la formule (3) suivante:
